(19) Europäisches Patentamt

European Patent Office

Office européen des brevets

(11) **EP 2 523 698 B1**

(12) **FASCICULE DE BREVET EUROPEEN**

(45) Date de publication et mention de la délivrance du brevet:
**03.12.2014 Bulletin 2014/49**

(21) Numéro de dépôt: **11704286.1**

(22) Date de dépôt: **13.01.2011**

(51) Int Cl.:
***A61L 15/58*** *(2006.01)*

(86) Numéro de dépôt international:
**PCT/FR2011/050063**

(87) Numéro de publication internationale:
**WO 2011/086330 (21.07.2011 Gazette 2011/29)**

(54) **NOUVEAU PANSEMENT COMPRENANT UN VOILE DE MICROFIBRES OU DE NANOFIBRES CAPABLE DE GELIFIER OU SE SOLUBILISER**

NEUE BANDAGE MIT EINEM NETZ AUS MIKROFASERN ODER NANOFASERN ZUR SCHNELLEN GELIERUNG ODER AUFLÖSUNG

NOVEL BANDAGE INCLUDING A WEB OF MICROFIBRES OR NANOFIBRES SUITABLE FOR GELLING OR SOLUBILISING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.01.2010 FR 1050222**

(43) Date de publication de la demande:
**21.11.2012 Bulletin 2012/47**

(73) Titulaire: **Laboratoires Urgo**
**21300 Chenove (FR)**

(72) Inventeur: **PERNOT, Jean-Marc**
**F-21000 Dijon (FR)**

(74) Mandataire: **Hubert, Philippe**
**Cabinet Beau de Loménie**
**158, rue de l'Université**
**75340 Paris Cedex 07 (FR)**

(56) Documents cités:
**EP-A1- 0 621 042      EP-A2- 1 020 198**
**WO-A1-99/27975      US-A- 5 681 305**

**Description**

La présente invention a pour objet un nouveau pansement repositionnable comprenant une masse hydrocolloïde adhésive destiné au traitement des plaies telles que les plaies exudatives, les brûlures, les lésions dermo-épidermiques superficielles, profondes, chroniques ou aïgues et en particulier au traitement de l'ampoule.

Les pansements comprenant des hydrocolloïdes sont connus depuis plus de 20 ans. Ils sont constitués d'un support sur lequel est déposée une masse adhésive comprenant des hydrocolloïdes. On peut citer, à titre d'exemples, les produits commercialisés sous les dénominations Algoplaque® par les Laboratoires URGO et Comfeel® par la société Coloplast. Des pansements comprenant une masse adhésive comprenant des hydrocolloïdes, spécifiquement destinés au traitement de l'ampoule, sont également connus et commercialisés par exemple sous les dénominations Urgo Traitement Ampoules® par les Laboratoires URGO et Compeed® par la société Johnson & Johnson.

Pour permettre une bonne absorption des exsudats de la plaie, ces pansements contiennent des quantités relativement importantes (de l'ordre de 20 à 50 % en masse) d'hydrocolloïdes. De manière préférentielle, ces pansements sont conçus pour tenir en place sans l'aide d'une bande adhésive complémentaire, en adhérant directement à la peau.

La masse adhésive de ces pansements connus est habituellement constituée d'une phase continue hydrophobe dans laquelle est dispersée une phase discontinue de particules d'hydrocolloïdes destinées à absorber les exsudats de la plaie.

L'absorption des exsudats par les hydrocolloïdes provoque la gélification de la masse adhésive, ce qui permet de retirer sans douleur le pansement de la plaie après son utilisation.

Pour assurer le maintien dans le temps de leur capacité d'absorption et de leur cohésion lors du retrait, ces pansements possèdent un pouvoir adhésif initial important. Ceci est encore plus vrai pour les pansements destinés au traitement des ampoules qui doivent être positionnés dans des zones courbes ou difficiles d'accès et qui sont soumis à de fortes contraintes mécaniques lors de leur utilisation.

Pour augmenter le pouvoir adhésif de ces pansements, on a travaillé :

- d'une part sur la composition qualitative et quantitative des masses adhésives les constituant; et
- d'autre part sur la forme de ces pansements, notamment en leur donnant des bords biseautés.

De tels pansements et leurs compositions sont bien connus et décrits par exemple dans les documents EP 264 299, EP 503 029, EP 1 020 198 et FR 2 495 473.

Toutefois, en raison de leur pouvoir adhésif important, ces pansements ne peuvent être repositionnés aisément au moment de leur mise en place sur la peau du patient. En effet, le retrait de ces pansements est très douloureux car le pansement adhère à la plaie ou à l'ampoule, tant qu'il n'a pas absorbé les exsudats ce qui peut nécessiter un temps relativement long de l'ordre de 15 minutes ou plus.

Le repositionnement du pansement, lors de sa mise en place, est cependant très souvent nécessaire, par exemple lorsque la surface du corps sur laquelle ce pansement doit être appliqué n'est pas plane, et ce problème est bien connu des personnels soignants.

En outre, ces pansements sont d'autant plus difficiles à mettre en place qu'ils sont généralement très fins, et, dans le cas des pansements destinés au traitement de l'ampoule, de petites dimensions et qu'ils doivent être positionnés dans des endroits difficiles d'accès : orteils, voûtes plantaires, talons, etc.

De nombreux utilisateurs ont déjà été confrontés à ce problème de repositionnement, en particulier dans le cas des pansements destinés au traitement de l'ampoule que l'on souhaite également pouvoir repositionner pour des raisons de discrétion et d'esthétique, par exemple pour éviter la formation de plis ou pour le dissimuler derrière la lanière d'une chaussure.

Si de nombreux systèmes applicateurs ont été développés depuis vingt ans pour faciliter la pose de ces pansements, ces systèmes ne permettent cependant pas de résoudre de manière satisfaisante le problème du repositionnement, et en particulier il n'existe pas à ce jour de solution idéale au problème du retrait sans douleur d'un pansement adhésif immédiatement après une première mise en place.

Le document EP 0 621 042 décrit un pansement hydrocolloïde dont la couche adhésive comprend un réseau tridimensionnel constitué de filaments polymériques revêtus d'une matière adhésive insoluble dans l'eau et les reliant entre eux à l'intérieur duquel sont dispersés des hydrocolloïdes. Ce réseau polymérique est destiné à maintenir l'intégrité et la cohésion du pansement même lorsque celui-ci a absorbé une quantité importante de liquide.

Le problème du retrait sans douleur du pansement immédiatement après sa pose, afin de permettre son repositionnement, tout en conservant ses propriétés d'adhésion, de cohésion et d'absorption au cours du temps, n'est d'aucune façon évoqué dans ce document antérieur.

Dans ces conditions, la présente invention a pour but de résoudre le nouveau problème technique consistant en la fourniture d'un pansement hydrocolloïde adhésif d'une nouvelle conception, qui puisse être retiré sans douleur immédiatement après sa pose pour être repositionné une ou plusieurs fois, tout en conservant ses propriétés d'adhésion, de cohésion et d'absorption au cours du temps.

Il a été découvert, et ceci constitue le fondement de la présente invention, qu'il était possible de résoudre ce problème technique, d'une façon relativement simple et utilisable à l'échelle industrielle, en modifiant la surface destinée à venir au contact de la peau d'un pansement adhésif hydrocolloïde traditionnel en y déposant un voile de microfibres ou de nanofibres aptes à se solubiliser ou gélifier au contact des exsudats de la plaie sensiblement totalement et en une durée très courte de préférence inférieure à 10 secondes, et de préférence encore inférieure à 1 seconde.

Ainsi, selon un premier aspect, la présente invention a pour objet un pansement comprenant une masse adhésive comprenant des hydrocolloïdes caractérisé en ce qu'il comprend, sur une partie au moins de la surface de la masse adhésive destinée à venir au contact de la plaie en position d'utilisation, un voile de microfibres ou de nanofibres constituées pour 90 % au moins, et de préférence 95 % au moins d'entre elles d'un (ou plusieurs) matériau(x) :

- choisi(s) parmi l'alcool polyvinylique (PVA);
  la poly (vinyl pyrrolidone) (PVP);
  le polyéthylènimine(PEI);
  les oxydes de polyéthylène(PEO);
  la carboxyméthylcellulose,
  les alginates ;

et les mélanges de ces composés ; et

- apte(s) à se solubiliser ou gélifier en moins de 10 secondes et de préférence encore en moins de 1 seconde au contact des exsudats de la plaie.

Comme on le comprend, en se solubilisant ou en gélifiant rapidement au contact des exsudats de la plaie, le voile de microfibres ou de nanofibres précité va former une couche intermédiaire non adhérente à la plaie immédiatement (quelques secondes au plus) après la première mise en place du pansement en permettant ainsi, en cas de besoin, de retirer le pansement de la plaie sans douleur et de le repositionner.

Il a été constaté que malgré la présence de ce voile de microfibres ou de nanofibres hydrosolubles ou gélifiables, le pansement conserve le même profil d'adhésion au retrait, les mêmes propriétés d'absorption qu'un pansement de même constitution mais ne comprenant pas ces microfibres ou nanofibres. En particulier, il n'a pas été observé, avec ces nouveaux pansements, de migration des exsudats au-delà ou autour de la partie de la surface de la masse adhésive recouverte par la couche formée après gélification ou solubilisation du voile de microfibres ou de nanofibres qui aurait pu altérer les propriétés adhésives ou les propriétés d'absorption et de cohésion du pansement en créant un différentiel d'absorption entre ces deux zones.

Selon une caractéristique particulière, ce voile peut contenir des particules de substances actives qui seront rapidement relarguées et qui faciliteront la cicatrisation de l'ampoule ou de la plaie.

Le pansement selon la présente invention peut être obtenu aisément, par simple dépose de microfibres ou de nanofibres, sur la surface destinée à venir au contact de la plaie d'un pansement adhésif contenant des hydrocolloïdes.

Les masses adhésives contenant des hydrocolloïdes susceptibles d'être utilisées pour la fabrication du pansement conforme à l'invention sont celles habituellement utilisées par l'homme de l'art pour la fabrication de pansements adhésifs hydrocolloïdes.

D'une façon générale, les masses adhésives des pansements selon l'invention présentent une adhésivité d'au moins 150 cN/ cm, et de préférence comprise entre 200 cN/cm et 8 N/cm mesurée sur plaque d'acier selon la norme européenne EN 1939 (mesure à 90°, vitesse de 100 mm/ min).

D'une façon générale, ces masses adhésives seront constituées d'une matrice élastomérique contenant des particules hydrocolloïdes et un (ou plusieurs) composé(s) destiné(s) à conférer des propriétés d'adhérence à ladite masse connu(s) sous le nom de « tackifiant(s) ».

Par « matrice élastomérique », on entend désigner ici une composition comprenant un (ou plusieurs) élastomère(s) choisi(s) parmi les copolymères séquencés poly(styrène-oléfine-styrène), dont les séquences oléfines peuvent être constituées d'unités isoprène, butadiène, éthylène-butylène, éthylène-propylène et leurs mélanges.

Dans le cadre de la présente invention, on préférera les copolymères triblocs poly(styrène-isoprène-styrène) (en abrégé : poly(SIS)) et les mélanges de copolymères triblocs poly(SIS) et de copolymères diblocs poly(styrène-isoprène), et en particulier les poly(SIS) ayant une teneur en styrène comprise entre 14 et 52 % et de préférence entre 14 et 30 % en poids rapporté au poids dudit poly(SIS).

De tels produits bien connus de l'homme de l'art sont par exemple commercialisés par la société Kraton sous la dénomination KRATON®D ou par la société Dexco Polymers LP sous la dénomination VECTOR®.

Parmi les copolymères triblocs poly(SIS) préférés, on peut citer en particulier les produits commercialisés sous les dénominations KRATON®D-1111CS, KRATON®D-1107 ou KRATON®1161, VECTOR®4114 et VECTOR®4113.

Des copolymères triblocs poly(styrène-butadiène-styrène) peuvent également être utilisés dans le cadre de l'invention.

Parmi ces copolymères poly(styrène-butadiène-styrène), on peut citer en particulier le produit commercialisé sous la dénomination KRATON®D-1102 par la société Kraton.

De préférence, les élastomères formant la matrice élastomérique seront présents, au sein de la masse adhésive des pansements selon l'invention en une quantité de 10 à 30 % en poids, et de préférence de 15 à 25 % en poids, du poids total de la masse adhésive.

D'une façon générale, la matrice élastomérique précitée incorpore un (ou plusieurs) hydrocolloïde(s).

Par «hydrocolloïde» on entend désigner ici tout composé habituellement utilisé par l'homme de l'art pour son aptitude à absorber les liquides hydrophiles tels que l'eau, le sérum physiologique ou les exsudats d'une plaie.

A titre d'exemple d'hydrocolloïde susceptible d'être utilisé dans le cadre de l'invention, on peut citer la pectine, les alginates, les gommes végétales naturelles comme en particulier la gomme de Karaya, les dérivés de cellulose comme en particulier les carboxyméthylcelluloses et leurs sels de métal alcalin, notamment de sodium ou de calcium, ainsi que les polymères synthétiques à base de sels de l'acide acrylique, connus sous l'appellation "superabsorbants", comme par en particulier les produits commercialisés par la société BASF sous la dénomination LUQUASORB® 1003 ou par la société CIBA Specialty chemicals sous la dénomination SALCARE® SC91. Bien entendu, des mélanges de ces produits peuvent être utilisés à titre d'hydrocolloïdes.

Il est à noter que ces composés sont présents au sein de la masse élastomérique et bien que susceptibles de se gélifier au contact des exsudats, leur éventuelle gélification n'est susceptible de se produire qu'après un lapse de temps relativement long, pouvant atteindre plusieurs heures, de sorte que ces composés ne sont pas susceptibles de résoudre le problème du repositionnement du pansement.

Les hydrocolloïdes préférés dans le cadre de la présente invention sont les sels de métal alcalin de la carboxyméthylcellulose, et en particulier la carboxyméthylcellulose de sodium.

La quantité d'hydrocolloïde(s) incorporée dans la matrice élastomérique sera adaptée en fonction du niveau d'absorption souhaité. D'une façon générale, la quantité d'hydrocolloïde(s) pourra être de l'ordre de 2 à 50 % en poids, rapportée au poids total de la masse adhésive.

Dans le cadre de la présente invention, on utilisera de préférence une quantité d'hydrocolloïde(s) comprise entre 20 et 50 % en poids rapportée au poids total de la masse adhésive si l'on désire réaliser un pansement absorbant du type de ceux décrits dans les documents EP 1 061 965, EP 1 165 717 et EP 0 927 051.

La matrice élastomérique contenant des particules hydrocolloïdes est rendue adhésive par l'ajout de produits dits « tackifiants » tels que ceux qui sont habituellement utilisés par l'homme de l'art dans la préparation des adhésifs sensibles à la pression à base d'élastomères et en particulier de copolymères séquencés poly(styrène-oléfine-styrène). Pour une description détaillée de ces produits, on pourra se reporter aux documents de l'état de la technique mentionnés précédemment ou à l'ouvrage de Donatas Satas "Handbook of Pressure Sensitive Technology", 3rd Edition, 1999, pages 346 à 398.

D'une façon générale, on pourra utiliser un (ou plusieurs) produit(s) tackifiant(s) qui sera (seront) incorporés(s) à la masse hydrocolloïde dans une large proportion de l'ordre de 1 à 70 % en poids rapportée au poids total de la masse adhésive, qui sera déterminée en fonction de la nature et de la proportion relative des autres constituants de cette dernière, pour obtenir le pouvoir adhésif souhaité pour la composition finale.

De préférence, le(s) produit(s) tackifiant(s) représentera (représenteront) de 10 à 40 % en poids, du poids total de la masse hydrocolloïde.

Les produits tackifiants susceptibles d'être utilisés dans le cadre de la présente invention pourront être choisis parmi les résines tackifiantes, les polyisobutylènes de bas poids moléculaire, les polybutènes de bas poids moléculaire ou leurs mélanges.

Parmi les résines tackifiantes susceptibles d'être utilisées selon l'invention, on peut mentionner les résines polyterpènes ou terpènes modifiées, les résines de colophane, les résines hydrocarbonnées, les mélanges de résine cyclique, aromatique et aliphatique, etc., ou des mélanges de ces résines.

De tels produits sont commercialisés par exemple :

- par la société GOODYEAR sous la dénomination WINGTACK®, comme en particulier la résine de synthèse formée de copolymères en C5/C9 (WINGTACK® 86) ou la résine à base de polyterpène synthétique (WINGTACK® 10) ;
- ou encore par la société HERCULES sous la dénomination KRISTALEX® comme en particulier la résine à base d'alphaméthylstyrène (KRISTALEX® 3085).

Ces résines tackifiantes peuvent être utilisées seules ou en mélange avec d'autres tackifiants, de préférence dans une proportion de 20 à 50 % en poids, et plus particulièrement de 25 à 35 % en poids, rapportée au poids total de la masse adhésive.

Parmi les polybutènes de bas poids moléculaire susceptibles d'être utilisés selon l'invention, on peut citer par exemple les produits commercialisés par la société BP CHIMIE sous la dénomination NAPVIS® et en particulier le produit commercialisé sous la dénomination NAPVIS® 10.

Ces polybutènes peuvent être utilisés seuls ou en mélange avec d'autres tackifiants, de préférence dans une proportion de 5 à 30 % en poids, et plus particulièrement de 8 à 15 % en poids, rapportée au poids total de la masse adhésive.

Parmi les polyisobutylènes de bas poids moléculaire susceptibles d'être utilisés selon l'invention, on peut citer les polyisobutylènes ayant un poids moléculaire de l'ordre de 40 000 à 80 000 daltons, comme par exemple les produits commercialisés par la société BASF sous la dénomination OPPANOL® et en particulier les produits commercialisés sous les dénominations OPPANOL®B12 et OPPANOL®B15.

Ces polyisobutylènes pourront être utilisés seuls ou en mélange avec d'autres tackifiants, de préférence dans une proportion de 5 à 30 % en poids, et plus particulièrement de 8 à 15 % en poids, rapportée au poids total de la masse adhésive.

Divers composés additionnels pourront être ajoutés à la matrice élastomérique contenant les composés tackifiants et hydrocolloïdes précités pour obtenir des masses adhésives hydrocolloïdes qui présentent des propriétés d'élasticité, d'adhésion, de stabilité dans le temps et de cohésion optimisées.

De tels composés sont par exemple des stabilisants comme en particulier des antioxydants, des plastifiants comme en particulier les polybutènes ou les huiles plastifiantes, ou des agents permettant d'améliorer la cohésion comme en particulier des caoutchoucs butyle ou des polyisobutylènes de haut poids moléculaire.

Parmi les polyisobutylènes de haut poids moléculaire susceptibles d'être utilisés selon l'invention pour améliorer la cohésion de la masse adhésive, on peut citer les polyisobutylène ayant un poids moléculaire de l'ordre de 400 000 à 2 000 000 daltons, comme par exemple les produits commercialisés par la société BASF sous les dénominations Oppanol®B12 SFN ou Oppanol®B30 SF.

Ces polyisobutylènes de haut poids moléculaire pourront être utilisés seuls ou en mélange, de préférence dans une proportion de 2 à 20 % en poids, et plus particulièrement de 5 à 15 % en poids, rapportée au poids total de la masse adhésive.

Par « stabilisant » on entend désigner ici tout composé apte à assurer la stabilité vis-à-vis de l'oxygène (antioxydant), la chaleur, l'ozone et les rayonnements ultra violets des composés utilisés dans la formulation des masses hydrocolloïdes adhésives, en particulier des résines tackifiantes et des copolymères séquencés. Ces composés stabilisants sont bien connus et pourront être utilisés seuls ou en mélange.

Parmi les composés antioxydants susceptibles d'être utilisés selon l'invention, on peut citer les antioxydants phénoliques, comme par exemple les produits commercialisés par la société CIBA-GEIGY sous les dénominations IRGANOX®1010, IRGANOX®565 et IRGANOX® 1076 ainsi que les antioxydants soufrés, comme par exemple le dibutyldithiocarbamate de zinc commercialisé par la société AKZO sous la dénomination PERKACIT®ZDBC.

Ces composés peuvent être utilisés seuls ou en mélange, de préférence dans une proportion de 0 à 2 % en poids, et plus particulièrement de 0,1 à 0,6 % en poids, rapportée au poids total de la masse hydrocolloïde.

Dans le cadre de la présente invention, on utilisera de façon préférée l'association de l'IRGANOX®1010 et du PERKACIT®ZDBC.

Parmi les composés plastifiants susceptibles d'être utilisés selon l'invention, on peut citer les plastifiants habituellement utilisés par l'homme de l'art pour la préparation de masses adhésives hydrocolloïdes et en particulier les polybutènes comme par exemple les produits commercialisés par la société BP CHEMICALS sous la dénomination NAPVIS® 10, les huiles plastifiantes ou encore des dérivés de phtalate tels que le dioctylphtalate.

L'utilisation des huiles plastifiantes est particulièrement préférée dans le cadre de la présente invention.

Par « huile plastifiante » on entend désigner ici les huiles minérales ou végétales couramment employées par l'homme de l'art pour plastifier les copolymères séquencés du type styrène-oléfine-styrène utilisés dans la composition des masses adhésives hydrocolloïdes.

Ces huiles minérales sont généralement constituées de mélanges dans des proportions variables de composés de nature paraffinique, naphténique ou aromatique.

Parmi les huiles plastifiantes susceptibles d'être utilisées selon l'invention, on peut citer les produits commercialisés par la société SHELL sous les dénominations ONDINA® et RISELLA® qui sont constitués de mélanges à base de composés naphténiques et paraffiniques ou sous la dénomination CATENEX® qui sont constitués de mélanges à base de composés naphténiques, aromatiques et paraffiniques.

Dans le cadre de la présente invention, on utilisera de façon préférentielle l'huile plastifiante minérale commercialisée sous la dénomination ONDINA®68.

Ces composés plastifiants peuvent être utilisés seuls ou en mélange, de préférence dans une proportion de 5 à 20 % en poids, et plus particulièrement de 7 à 15 % en poids, rapportée au poids total de la masse hydrocolloïde.

La masse adhésive hydrocolloïde des pansements selon l'invention peut encore comprendre un ou plusieurs composé(s) tensioactif(s) en une quantité inférieure ou égale à 10 % en poids, de préférence inférieure ou égale à 5 % en poids, rapportée au poids total de la masse hydrocolloïde.

Un composé tensioactif préféré dans le cadre de la présente invention est le composé commercialisé sous la dénomination AcResin®.

Un autre composé tensioactif préféré dans le cadre de la présente invention est le polysorbate 80, comme par exemple le produit commercialisé par la société SEPPIC sous la dénomination MONTANOX® 80.

Dans le cadre de la présente description, par l'expression « voile de microfibres ou de nanofibres » on entend désigner un ensemble de microfibres ayant de préférence un diamètre compris entre 1 et 50 $\mu$m, de préférence encore compris entre 1 et 25 $\mu$m et/ou de nanofibres ayant un diamètre compris entre 20 et 1 000 nm, et de préférence encore compris entre 50 et 500 nm disposées, de préférence selon une disposition monocouche, en formant un voile analogue à un non-tissé, c'est à dire un assemblage aléatoire de microfibres et/ou nanofibres maintenu sous forme d'une nappe par friction, cohésion ou adhésion.

Les voiles de microfibres ou de nanofibres selon l'invention peuvent être obtenus par étirage aérodynamique de polymères fondus ou en solution aqueuse ou solvant selon la technologie connue sous le nom de "centrifugal spinning".

Les voiles de microfibres ou de nanofibres peuvent également être obtenus par le procédé connu sous le nom d'"'électrofilage" ou "electrospinning".

L'électrofilage est une technologie permettant la réalisation de nanofibres par évaporation d'une solution ou d'une dispersion de polymère au sein d'un champ électrique à potentiel élevé. Plus précisément, ce procédé consiste à soumettre une solution ou dispersion d'un matériau suffisamment fluide, sortant d'une buse très fine à un potentiel électrique de l'ordre de 5 à 50 Kv. Sous l'effet de ce champ électrique, la goutte sortant de la buse se charge électriquement en prenant une forme sensiblement conique et peut être étirée en jet pour former une fibre très fine de taille nanométrique à micrométrique lorsque la tension électrique est suffisamment élevée pour rompre la tension superficielle de la goutte. Les fibres ainsi formées peuvent être recueillies et conservées en l'état par exemple sur un collecteur, ou bien encore déposées sur un support de façon aléatoire pour former un voile semblable à un non tissé. Cette technologie est particulièrement adaptée à la préparation de microfibres ou nanofibres à base d'alcool polyvinylique.

Quelle que soit la technologie utilisée, le voile de microfibres ou de nanofibres peut être formé préalablement puis déposé sur la surface de la masse adhésive hydrocolloïde. Alternativement, le voile peut être formé "in situ" en utilisant la surface de la masse adhésive hydrocolloïde comme support lors de la formation du voile

Comme on le comprend, les microfibres ou nanofibres utilisées dans le cadre de la présente invention sont des produits connus.

D'une façon générale, les microfibres ou nanofibres formant le voile sont constituées pour 90 % au moins, et de préférence pour 95 % au moins d'entre elles, d'un ou plusieurs matériaux choisis parmi les polymères naturels ou synthétiques, et apte à se solubiliser ou gélifier en moins de 10 secondes et de préférence encore en moins de 1 seconde au contact des exsudats de la plaie.

Par l'expression « matériau apte à se solubiliser» on entend désigner tout matériau capable de se dissoudre dans les exsudats de la plaie en formant une solution homogène.

Par l'expression « matériau apte à gélifier » on entend désigner tout matériau capable de former un gel au contact des exsudats de la plaie, ie une substance présentant une viscosité supérieure à celle de l'eau, par exemple supérieure à $10^{-3}$ Pa.s et de préférence supérieure à $10^{-2}$ Pa.s.

Parmi les matériaux susceptibles d'être utilisés selon l'invention, on peut citer les polymères hydrosolubles ou hydrodispersibles et en particulier :

l'alcool polyvinylique (PVA);
la poly (vinyl pyrrolidone) (PVP);
le polyéthylènimine(PEI);
les oxydes de polyéthylène(PEO);
la carboxyméthylcellulose,
les alginates ;

et les mélanges de ces composés.

D'excellents résultats ont été obtenus en utilisant des microfibres ou nanofibres d'alcool polyvinylique qui constitue donc un matériau préféré pour la mise en oeuvre de l'invention.

Les microfibres ou nanofibres utilisées dans le cadre de la présente invention peuvent être de différentes natures, en fonction du degré de solubilité, de la résistance mécanique ou de la résistance à la température désirés.

Selon un mode de réalisation particulier de l'invention, le voile de microfibres ou nanofibres contient une (ou plusieurs) substance(s) active(s).

Les substances actives susceptibles d'être utilisées dans le cadre de la présente invention peuvent être choisies parmi les antifongiques, les anti-microbiens ou antibactériens tels que la sulfadiazine argentique, les régulateurs de pH, les accélérateurs de cicatrisation tels que l'acide hyaluronique, les vitamines, les agents hydratants, les oligo-éléments, les anesthésiques locaux, les piégeurs d'odeur, le menthol, le salicylate de méthyle, les hormones, les anti-inflammatoires et les mélanges de ces composés.

Une substance active préférée dans le cadre de l'invention est l'acide hyaluronique.

Les substances actives pourront être incorporées au voile soit avant ou après que celui-ci ait été formé à la surface de la masse adhésive hydrocolloïde, par tout moyen permettant leur dépôt. Alternativement, ces substances actives pourront être incorporées au microfibres ou nanofibres avant que celles-ci soient déposées sous forme de voile à la surface de la masse adhésive hydrocolloïde.

Dans le cas où le voile de microfibres ou de nanofibres est préparé par électrofilage, les substances actives pourront être incorporées dans la solution ou dispersion soumise au champ électrique, (par exemple dans une solution d'alcool polyvinylique dans le cas de microfibres ou nanofibres d'alcool polyvinylique) en permettant ainsi d'intégrer ces substances actives directement dans le voile de microfibres ou de nanofibres.

Selon l'invention les microfibres ou nanofibres sont disposées, de façon à former un voile à la surface de la masse adhésive hydrocolloïde destinée à venir en contact avec la plaie. En d'autres termes, ces microfibres ou nanofibres ne seront pas présentes à l'intérieur de la masse adhésive hydrocolloïde.

D'une façon générale, le voile formé de ces microfibres ou nanofibres recouvrira entre 5 et 90 %, et de préférence entre 10 et 60%, de la surface de la masse adhésive venant au contact de la plaie, selon l'application envisagée. Ainsi, le taux de recouvrement de la surface de la masse adhésive venant au contact de la plaie sera de l'ordre de 10% à 50% pour un pansement destiné au traitement de l'ampoule et de l'ordre de 20 % à 70 % pour un pansement destiné au traitement de l'escarre ou de l'ulcère.

Les microfibres ou nanofibres pourront être incorporées au pansement conforme à l'invention par tout procédé permettant leur dépôt à la surface de la masse adhésive hydrocolloïde sous forme de voile.

Par exemple, cette opération peut être avantageusement réalisée par simple dépose d'un voile préalablement constitué ou par la mise en oeuvre des procédés d'électrofilage ou de centrifugal spinning décrit précédemment.

Selon un mode de réalisation préféré de l'invention, les microfibres ou nanofibres seront disposées uniquement au niveau de la zone du pansement destinée à venir en contact avec la zone de la plaie à traiter.

Par exemple, les microfibres ou nanofibres pourront être déposées au niveau des zones destinées à constituer la partie centrale des pansements.

Les microfibres ou nanofibres pourront être déposées selon une forme géométrique quelconque, par exemple une forme carrée, rectangulaire, ou ovale, ajourée ou non. La quantité de microfibres ou nanofibres déposée peut varier dans de larges proportions et sera généralement comprise entre 1 et 100 g, et de préférence entre 1 et 30 g par mètre carré de masse adhésive.

Les pansements de l'invention peuvent être de différents types mais comprendront de préférence un support.

La masse adhésive portant le voile de microfibres ou nanofibres peut être complexée à un support du type de ceux utilisés dans les pansements commercialisés par la société Laboratoires Urgo sous les dénominations Algoplaque®, ou par la société Convatec sous la dénomination Duoderm® ou encore par la société Coloplast sous la dénomination Comfeel®. De tels pansements sont décrits dans les documents FR 2 392 076, FR 2 495 473, WO 98/10801 et EP 264 299.

D'une façon générale, le choix du support sera réalisé en fonction des propriétés requises (étanchéité, élasticité, etc.) dans l'application recherchée.

Ainsi, le pansement selon l'invention peut comprendre un support tel qu'un film formé d'une ou plusieurs couches et d'une épaisseur variable de 5 à 150 $\mu$m ; un non-tissé ou encore une mousse ayant une épaisseur de 10 à 500 $\mu$m sur lequel la masse adhésive hydrocolloïde a été enduite, de façon continue ou non continue.

Ces supports à base de matériaux synthétiques ou naturels sont bien connus de l'homme de l'art.

Parmi les supports en forme de mousse utilisables dans le cadre de l'invention, on peut ainsi citer les mousses en polyéthylène, en polyuréthane, en PVC par exemple.

Parmi les supports non-tissés utilisables dans le cadre de l'invention, on peut citer les non-tissés en polypropylène, polyéthylène, polyuréthane, polyamide, ou polyester par exemple.

Dans le cadre de la présente invention, on préférera utiliser des supports en forme de films, et notamment des films en polyuréthanne comme par exemple les films commercialisés par la société Smith et Nephew sous la référence LASSO® réalisés à partir du polyuréthanne commercialisé par la société BF GOODRICH sous la dénomination ESTANE® ; des films en polyéthylène à basse densité comme par exemple les films commercialisés par la société SOPAL ; des films à base de copolymère thermoplastique de polyétherpolyester comme par exemple les produits commercialisés par la société DUPONT DE NEMOURS sous la dénomination Hytrel® ; ou encore des films complexes associant un film de polyuréthanne et un non-tissé.

Selon une variante de réalisation de l'invention, le pansement peut comporter une couche absorbante disposée entre le support et la masse hydrocolloïde adhésive. Cette couche absorbante peut être constituée de tout type de matériau absorbant tel que par exemple une mousse (comme en particulier une mousse polyuréthane), un non-tissé, une couche de polymère super absorbant, ou une combinaison de ces matériaux.

Les pansements réalisés à partir de la masse adhésive hydrocolloïde selon l'invention peuvent se présenter sous une forme géométrique quelconque, par exemple carrée, rectangulaire, circulaire ou ovale. De même leur taille peut être quelconque et sera adaptée en fonction de la surface de la partie à traiter ou protéger. Par exemple, un pansement destiné au traitement de l'ampoule se présentera sous une forme rectangulaire d'environ 7 cm de longueur et 4 cm de largeur tandis qu'un pansement destiné au traitement de l'ulcère se présentera sous une forme carrée de 10 cm de coté.

De façon pratique, la surface de la masse adhésive hydrocolloïde portant le voile de microfibres ou nanofibres pourra être recouverte d'un film ou pellicule de protection destiné à être retiré, par exemple par pelage, avant la mise en place du pansement sur la plaie et/ou sur la peau.

L'ensemble ainsi formé pourra être lui-même emballé dans une protection étanche formée par exemple au moyen de complexes polyéthylène-aluminium ou dans des blisters.

L'invention sera illustrée par les exemples non limitatifs suivants.

## EXEMPLE 1 : Préparation d'un pansement hydrocolloïde standard

On a préparé une masse adhésive hydrocolloïde constituée des composés suivants (quantité exprimée en poids pour 100 grammes de masse).

| N° | Composés | Quantité (en poids pour 100 grammes) |
|---|---|---|
| 1 | Elastomère<br>Copolymère bloc Styrène-Isoprène-Styrène/ Styrène Isoprène (commercialisé sous la dénomination Vector® 4114A par Dexco Polymers LP) | 14,144 |
| 2 | Agent améliorant la cohésion<br>Polyisobutène (commercialisé sous la dénomination Oppanol® B12SFN par BASF) | 3,536 |
| 3 | Plastifiant<br>Huile minérale (commercialisé sous la dénomination Ondina® 933 par Shell) | 12,376 |
| 4 | Tensioactif<br>Copolymère ester acrylique réticulable UV (commercialisé sous la dénomination AcResin® A258UV par BASF) | 6,542 |
| 5 | Résine tackifiante<br>Résine synthétique d'hydrocarbures (commercialisé sous la dénomination de Wingtack® 86 par Safic Alcan) | 26,521 |
| 6 | Hydrocolloïde<br>Carboxyméthylcellulose (commercialisé sous la dénomination CMC Blanose® 7H4XF par Hercules) | 35,714 |
| 7 | Tensioactif<br>Polysorbate 80 (commercialisé sous la dénomination Montanox® 80 par la société SEPPIC) | 0,460 |
| 8 | Antioxydant<br>Dibutyldithiocarbamate de Zinc (commercialisé sous la dénomination Perkacit® ZDBC par la société FLEXSYS (distributeur ARNAUD)) | 0,354 |
| 9 | Antioxydant<br>Pentaérythritol Tetrakis (3-3,5-di-tert-butyl-4-hydroxyphényl)proprionate) (commercialisé sous la dénomination Irganox® 1010 par Ciba Speciality Chemicals) | 0,354 |

Cette masse adhésive hydrocolloïde a été préparée par la mise en oeuvre du procédé suivant :

Dans un malaxeur à bras en Z à une température de consigne de 140°C, on a introduit les composés 1, 2, 3, 8 et 9.
A la 30ème minute, on a ajouté le composé 4.
A la 40ème minute, on a ajouté le composé 7.
A la 45ème minute, on a ajouté le composé 5.

A la 60<sup>ème</sup> minute, on a ajouté le composé 6.

La vidange du malaxeur a été effectuée à la 80<sup>ème</sup> minute.

La masse hydrocolloïde adhésive a été chauffée à une température de l'ordre de 110 à 130°C, puis enduite sur un support constitué d'un film en polyuréthane ayant une épaisseur de 30 $\mu$m. Le complexe formé de la masse hydrocolloïde et du support a ensuite été découpé en pansements individuels, couverts, au niveau de la surface hydrocolloïde adhésive libre, d'ailettes de protection en polyester siliconé.

## EXEMPLE 2 : Préparation d'un pansement selon l'invention

La masse hydrocolloïde adhésive préparée à l'exemple 1 a été chauffée à une température de l'ordre de 110 à 130°C, puis enduite sur un support constitué d'un film en polyuréthane ayant une épaisseur de 30 $\mu$m. Le complexe formé par la masse adhésive hydrocolloïde et le film en polyuréthane a été découpé en pansements individuels.

On a déposé ensuite sur la surface libre (non liée au support) de la masse adhésive hydrocolloïde un voile préalablement formé par électrofilage de nanofibres à base d'alcool polyvinylique (produit commercialisé par la société SEPPIC sous la dénomination Mowiol 40-88) ayant un diamètre de 100 à 200 nm.

Ces nanofibres ont été déposées au centre du pansement par simple dépose du voile de nanofibres en une quantité comprise entre 10 et 15 g/m$^2$.

La surface de la masse hydrocolloïde adhésive portant les nanofibres a été couverte d'ailettes de protection en polyester siliconé.

## EXEMPLE 3: Mise en évidence des propriétés du pansement conforme à l'invention

On a mesuré l'adhésivité des pansements tel que préparés aux exemples 1 et 2, après contact avec du sérum physiologique pour simuler l'exsudation d'une plaie, afin d'évaluer la différence de comportement au retrait entre un pansement comprenant une masse adhésive hydrocolloïde traditionnelle (exemple 1) et un pansement selon l'invention comprenant une masse adhésive hydrocolloïde dont la surface destinée à venir au contact de la peau porte un voile de nanofibres (exemple 2).

A cet effet, on a utilisé la méthode dite du "tack à la sonde" qui vise à mesurer le décollement d'une sonde cylindrique appliquée sur un adhésif avec une contrainte de pression $P_0$ pendant un temps t donné dans des conditions de température et d'humidité prédéterminées.

### Appareillage

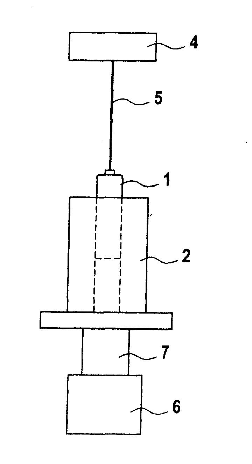

Plus précisément, cette méthode a été mise en oeuvre au moyen de l'appareil représenté à la figure 1.

Cet appareil est essentiellement constitué :

- d'une sonde cylindrique 1 présentant une extrémité finement polie susceptible de se déplacer longitudinalement (de bas en haut dans l'exemple représenté) au travers d'un guide sonde 2 constitué d'un cylindre percé en son centre d'un trou libre permettant le libre passage sans frottement de la sonde;
- d'un dynamomètre électronique comprenant une mâchoire mobile 4 pouvant être reliée à la sonde 1 par une chaînette 5 et un socle 6 sur lequel peut être emboîté un plateau 7 ; ledit dynamomètre étant en outre relié à un système d'acquisition et d'enregistrement des données telles qu'en particulier la vitesse de descente de la sonde, la pression d'accostage de la sonde sur l'adhésif, le temps de contact entre la sonde et l'adhésif et la vitesse de remontée de la sonde.

### Echantillons

### Pansement standard

En suivant le protocole expérimental de l'exemple 1, on a préparé un pansement sensiblement carré de 20 cm de côté.

Ce pansement, a été recouvert d'un ruban adhésif double face sur la face formant support. Un rouleau presseur a été employé afin de bien faire adhérer ledit ruban à la surface du pansement.

Des échantillons circulaires de même surface que la sonde ont été découpés dans l'ensemble ainsi constitué par le pansement et le ruban adhésif précité à l'aide d'un emporte pièce circulaire de 30 mm de diamètre.

Pansement selon l'invention

Un second jeu d'échantillons de même nature et de même dimension a été préparé.

On a déposé sous forme de voile des nanofibres à base d'alcool polyvinylique telles que celles utilisées à l'exemple 2 sur la surface de ces échantillons.

Mode opératoire :

- On a emboîté le plateau 7 sur le socle 6 du dynamomètre.
- On a fixé la chaînette 5 de la sonde dans la mâchoire mobile 4 du dynamomètre.
- On a nettoyé la sonde 1 avec un solvant (cette opération étant répétée avant chaque mesure).
- On a appliqué un échantillon (coté double face) tel que préparé précédemment sur la sonde.
- On a déposé 50 $\mu$l de sérum physiologique sur le plateau 7 au milieu de la zone délimitée par le guide-sonde.
- On a programmé les paramètres du dynamomètre (vitesse de descente, pression d'accostage, temps de contact et vitesse de remontée).
- On a procédé à la mesure en effectuant les opérations suivantes: descente de la sonde, mise en contact de l'échantillon adhésif et du sérum physiologique.
- On a enregistré la force de décollement en kPa que l'on a traduite en contrainte d'arrachement C suivant la relation :

$$C = F/S$$

où F = Force exprimée en N et S= Surface exprimée en m$^2$.

Paramètres du test:

Contrainte d'application : 1.7kPa (sonde aluminium de 121g et diamètre de 30 mm)
Vitesse d'essai : 300 mm/min
Rouleau presseur : 4 kg
Temps de contact : 1 s

Les mesures ont été réalisées sur 3 échantillons de chacun des pansements standard et selon l'invention et répétées 6 fois.

Résultats :

Les résultats des mesures ainsi effectuées exprimés en kPa ont été reportés dans le tableau I.

TABLEAU I

| Essai N° | Exemple 1 | Exemple 2 |
|---|---|---|
| 1 | 138,8 | 0,2 |
| 2 | 182,8 | 0,3 |
| 3 | 171,2 | 0,6 |
| 4 | 160,7 | 0,6 |
| 5 | 134,4 | 0,9 |
| 6 | 194 | 0,7 |

Observations du comportement des pansements selon les exemples 1 et 2 en présence de sérum physiologique.

| Conditions | Observations après contact avec sérum physiologique |
|---|---|
| Exemple 1 + 50µl sérum physiologique | Gélification totale après 15 minutes |
| Exemple 2 +50µl sérum physiologique | Solubilisation en une seconde |

Conclusions

La solubilisation quasi totale du voile est très rapide (1s).

Le test du "tack à la sonde" a permis de constater que l'adhésivité au retrait d'un pansement adhésif hydrocolloïde selon l'invention est bien inférieure à celle d'un pansement standard sans voile.

Lorsque la masse adhésive hydrocolloïde du pansement est recouverte d'un voile de microfibres ou de nanofibres, l'adhésivité au retrait de celui-ci devient minime, voire même pratiquement nulle lorsqu'il rentre en contact avec du sérum physiologique, même en très faible quantité.

Ainsi, un pansement selon l'invention, c'est à dire comprenant une masse adhésive hydrocolloïde dont la surface destinée à venir en contact avec la plaie est recouverte au moins en partie de microfibres ou de nanofibres hydrosolubles ou gélifiables, pourra être facilement repositionné après une première application, et ce dans les secondes qui suivent ladite première application.

**Revendications**

1. Pansement comprenant une masse adhésive comprenant des hydrocolloïdes **caractérisé en ce qu'**il comprend, sur une partie au moins de la surface de la masse adhésive destinée à venir au contact de la plaie en position d'utilisation, un voile de microfibres ou de nanofibres constituées pour 90 % au moins, et de préférence 95 % au moins d'entre elles d'un (ou plusieurs) matériau(x) :

    - choisi(s) parmi l'alcool polyvinylique (PVA);
    la poly (vinyl pyrrolidone) (PVP);
    le polyéthylènimine(PEI);
    les oxydes de polyéthylène(PEO);
    la carboxyméthylcellulose,
    les alginates ;

    et les mélanges de ces composés; et

    - apte(s) à se solubiliser ou gélifier en moins de 10 secondes et de préférence encore en moins de 1 seconde au contact des exsudats de la plaie.

2. Pansement selon la revendication 1, **caractérisé en ce que** les microfibres précitées ont un diamètre compris entre 1 et 50 µm, de préférence entre 1 et 25 µm.

3. Pansement selon la revendication 1 ou 2, **caractérisé en ce que** les nanofibres précitées ont un diamètre compris entre 20 et 1 000 nm, et de préférence entre 50 et 500 nm.

4. Pansement selon l'une des revendications précédentes, caractérisé en ce les microfibres ou nanofibres précitées sont présentes en une quantité de 1 à 100 g, et de préférence de 1 à 30 g par m$^2$ de surface de masse adhésive.

5. Pansement selon l'une des revendications précédentes, **caractérisé en ce que** le voile de microfibres ou de nanofibres précité recouvre 10 à 60 % de la surface de la masse adhésive destinée à venir au contact de la plaie en position d'utilisation.

6. Pansement selon l'une des revendications précédentes, **caractérisé en ce que** le matériau constituant les microfibres ou nanofibres précité est l'alcool polyvinylique.

7. Pansement selon l'une des revendications précédentes, **caractérisé en ce que** le voile de microfibres ou nanofibres contiennent une ou plusieurs substance(s)active(s) choisie(s) parmi les accélérateurs de cicatrisation, les antifongiques, les anti-microbiens ou antibactériens, les agents hydratants, les anesthésiques locaux, les anti-inflammatoires ou un mélange de ces composés.

8. Pansement selon la revendication 7, **caractérisé en ce que** la substance active précitée est l'acide hyaluronique.

9. Pansement selon l'une des revendications précédentes, **caractérisé en ce qu'**il comprend un support.

10. Pansement selon l'une des revendications précédentes, **caractérisé en ce qu'**il comprend une couche absorbante située entre le support et la masse hydrocolloïde.


**Patentansprüche**

1. Verband, umfassend eine Hydrokolloide umfassende haftende Masse, **dadurch gekennzeichnet, dass** er auf wenigstens einem Teil der Fläche der haftenden Masse, die dazu bestimmt ist, in Gebrauchsposition mit der Wunde in Kontakt zu kommen, ein Vlies aus Mikrofasern oder aus Nanofasern umfasst, die zu wenigstens 90 % und vorzugsweise wenigstens 95 % unter sich von einem (oder mehreren) Material(ien) gebildet sind:

   - das (die) ausgewählt ist (sind) aus Polyvinylalkohol (PVA);
   Poly(vinylpyrrolidon) (PVP);
   Polyethylenimin (PEI);
   den Polyethylenoxiden (PEO);
   Carboxymethylcellulose;
   den Alginaten;

   sowie den Mischungen dieser Verbindungen; und

   - das (die) geeignet ist (sind), in weniger als 10 Sekunden und vorzugsweise weiterhin in weniger als 1 Sekunde bei Kontakt mit den Exsudaten der Wunde löslich zu werden oder zu gelieren.

2. Verband nach Anspruch 1, **dadurch gekennzeichnet, dass** die vorgenannten Mikrofasern einen Durchmesser im Bereich zwischen 1 und 50 $\mu$m, vorzugsweise zwischen 1 und 25 $\mu$m haben.

3. Verband nach Anspruch 1 oder 2, **dadurch gekennzeichnet, dass** die vorgenannten Nanofasern einen Durchmesser im Bereich zwischen 20 und 1000 nm und vorzugsweise zwischen 50 und 500 nm haben

4. Verband nach einem der vorhergehenden Ansprüche, **dadurch gekennzeichnet, dass** die vorgenannten Mikrofasern oder Nanofasern in einer Menge von 1 bis 100 g und vorzugsweise von 1 bis 30 g pro m$^2$ Fläche haftender Masse vorhanden sind.

5. Verband nach einem der vorhergehenden Ansprüche, **dadurch gekennzeichnet, dass** das vorgenannte Mikrofaser- oder Nanofaservlies 10 bis 60 % der Fläche der haftenden Masse, die dazu bestimmt ist, in Gebrauchsposition mit der Wunde in Kontakt zu kommen, bedeckt.

6. Verband nach einem der vorhergehenden Ansprüche, **dadurch gekennzeichnet, dass** das vorgenannte Material, das die Mikrofasern oder Nanofasern bildet, Polyvinylalkohol ist.

7. Verband nach einem der vorhergehenden Ansprüche, **dadurch gekennzeichnet, dass** das Mikrofaser- oder Nanofaservlies einen oder mehrere Wirkstoff(e) enthält, der (die) aus den Wundheilungsbeschleunigern, den Fungiziden, den antimikrobiellen Wirkstoffen oder antibakteriellen Wirkstoffen, den feuchtigkeitsspendenden Mitteln, den Lokalanästhetika, den entzündungshemmenden Mitteln oder einer Mischung dieser Verbindungen ausgewählt ist (sind).

8. Verband nach Anspruch 7, **dadurch gekennzeichnet, dass** der vorgenannte Wirkstoff Hyaluronsäure ist.

9. Verband nach einem der vorhergehenden Ansprüche, **dadurch gekennzeichnet, dass** er einen Träger umfasst.

**10.** Verband nach einem der vorhergehenden Ansprüche, **dadurch gekennzeichnet, dass** er eine zwischen dem Träger und der Hydrokolloidmasse befindliche absorbierende Schicht umfasst.

**Claims**

**1.** A wound dressing comprising an adhesive mass comprising hydrocolloids, **characterized in that** the dressing comprises, on a portion at least of the surface of the adhesive mass intended to come into contact with the wound in the position of use, a web of microfibers or nanofibers which are composed, for 90% at least and preferably 95% at least of them, of one (or more) material(s):

- chosen from polyvinyl alcohol (PVA);
poly(vinylpyrrolidone) (PVP);
polyethyleneimine (PEI);
polyethylene oxides (PEOs);
carboxymethylcellulose,
alginates;

and the mixtures of these compounds; and

- capable of dissolving or gelling in less than 10 seconds and more preferably in less than 1 second on contact with the exudates from the wound.

**2.** The wound dressing as claimed in claim 1, **characterized in that** the abovementioned microfibers have a diameter of between 1 and 50 $\mu$m, preferably between 1 and 25 $\mu$m.

**3.** The wound dressing as claimed in claim 1 or 2, **characterized in that** the abovementioned nanofibers have a diameter of between 20 and 1000 nm, preferably between 50 and 500 nm.

**4.** The wound dressing as claimed in one of the preceding claims, **characterized in that** the abovementioned microfibers or nanofibers are present in an amount of 1 to 100 g, preferably of 1 to 30 g, per $m^2$ of surface area of adhesive mass.

**5.** The wound dressing as claimed in one of the preceding claims, **characterized in that** the abovementioned web of microfibers or nanofibers covers from 10 to 60% of the surface of the adhesive mass intended to come into contact with the wound in the position of use.

**6.** The wound dressing as claimed in one of the preceding claims, **characterized in that** the abovementioned material constituting the microfibers or nanofibers is polyvinyl alcohol.

**7.** The wound dressing as claimed in one of the preceding claims, **characterized in that** the web of microfibers or nanofibers comprises one or more active substance(s) chosen from healing accelerators, antifungals, antimicrobials or antibacterials, moisturizing agents, local anesthetics, anti-inflammatories or a mixture of these compounds.

**8.** The wound dressing as claimed in claim 7, **characterized in that** the abovementioned active substance is hyaluronic acid.

**9.** The wound dressing as claimed in one of the preceding claims, **characterized in that** it comprises a backing.

**10.** The wound dressing as claimed in one of the preceding claims, **characterized in that** it comprises an absorbent layer situated between the backing and the hydrocolloid mass.

**EP 2 523 698 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 264299 A
- EP 503029 A
- EP 1020198 A
- FR 2495473
- EP 0621042 A
- EP 1061965 A
- EP 1165717 A
- EP 0927051 A
- FR 2392076
- WO 9810801 A

**Littérature non-brevet citée dans la description**

- **DONATAS SATAS.** Handbook of Pressure Sensitive Technology. 1999, 346-398